# EUROPEAN PATENT APPLICATION

(11) **EP 4 570 899 A1**
(43) Date of publication of application: **18.06.2025**
(21) Application number: 22782747.4
(22) Date of filing: 11.08.2022
(51) Int. Cl.: C12M 3/00, C12M 3/06

(54) **ORGAN-ON-CHIP DEVICE**

(71) Applicant: Medtech Innovation on Advanced Medicine S.L.U., 08225 Terrassa (Barcelona) (ES)
(72) Inventor: GOMBAU SUÁREZ, Lourdes, 08005 BARCELONA (ES); OLLÉ MONGE, Marta, 08005 BARCELONA (ES); CUCALA VELA, Jonatan, 08005 BARCELONA (ES); CECILIA PADILLA, David, 08005 BARCELONA (ES); CABOT CANYELLES, Joan Marc, 08005 BARCELONA (ES); DEL POZO FÁBREGAS, Blanca, 08005 BARCELONA (ES); AZIZIAN, Pooya, 08005 BARCELONA (ES)
(74) Representative: Herrero & Asociados, S.L.
(86) International application number: PCT/ES2022/070535
(87) International publication number: WO 2024/033549

(57) **Abstract**

The organ-on-chip device comprises a microfluidic plate (1), a base (2), and a plurality of independent subunits, each subunit comprising an upper channel (12) and a lower channel (13), said channels (12, 13) being defined between the microfluidic plate (1) and the base (2), wherein the microfluidic plate (1) has a wettability contact angle greater than 30°, and the base (2) has a wettability contact angle lower than 90°. The organ-on-chip device permits to separate the channels without using a membrane and having them one on top of the other.

## Description

### Field of the invention

The present invention relates to an organ-on-chip device that does not need the use of a membrane for separating the channels of the organ-on-chip device.

### Background of the invention

Organ-on-chip devices are microfluidic devices for culturing living cells in micrometer sized chambers in order to model physiological functions of tissues and organs. Engineered patterning and continuous fluid flow in these devices has allowed, e.g., culturing of intestinal cells bearing physiologically relevant features and sustained exposure to bacteria while maintaining cellular viability, thereby allowing study of inflammatory bowel diseases.

Organ-on-chip devices are usually formed by at least 2 regions interconnected comprising the perfusion channels and reservoirs. Sometimes, a membrane is used for separating the perfusion channels and obtaining a lower channel and an upper channel independent from each other. This membrane is usually made of polyester, and it allows to generate an independency among the channels of the upper and lower parts.

Furthermore, this membrane is needed to separate the channels, so that the user can work with two different and independent solutions and/or conditions (e.g., monolayer of cells, cells encapsulated in an extracellular matrix, organoids, perfusion media). However, an assembly like that supposes an extra cost to the manufacture of the device and makes the fabrication process longer and laborious.

There are also known organ-on-chip devices that use a phase-guide (or other obstacle such as pillars or columns) to separate the channels using a hydrogel. This phase-guide has the same function as the membrane, i.e., to separate the channels that conform the device. However, in this case, the channels are aside, so that cells grow perpendicularly, i.e., vertically, to the microscope view, so it is difficult to inspect cell growth.

### Disclosure of the invention

Therefore, an objective of the present invention is to provide an organ-on-chip device that permits to separate the channels without using a membrane between them and obtaining having them one on top of the other.

With the organ-on-chip device of the invention said disadvantages are solved, presenting other advantages that will be described below.

The organ-on-chip device according to the present invention is defined in claim 1. Other optional features are defined in the dependent claims.

In particular, the organ-on-chip device comprises a microfluidic plate, a base, and a plurality of independent subunits, each subunit comprising an upper channel and a lower channel, said channels being defined between the microfluidic plate and the base, the microfluidic plate having a wettability contact angle greater than 30°, and the base having a wettability contact angle lower than 90°.

According to two alternative embodiments, the microfluidic plate is made of poly(methyl methacrylate) and the wettability contact angle is between 60° and 75°, or the microfluidic plate is made of polydimethylsilozane and the wettability contact angle is between 100° and 110°.

According to two alternative embodiments, the base is made of polyester film and the wettability contact angle is between 15° and 25°, or the base is made of glass and the wettability contact angle is between 25° and 35°.

Advantageously, the material of the base is more hydrophilic than the material of the microfluidic plate.

Furthermore, the upper and lower channels of each subunit are overlapped to each other, preferably, directly overlapped, i.e., with no physical separation between them.

In the organ-on-chip device according to present invention, each of the lower and upper channels of each subunit define an area at the contact point to each other, the ratio of the area between the lower channel and the upper channel being between 0 and 0.98, such as e.g., between 0.15 and 0.30.

Preferably, each channel is connected to two cylindric chambers to be used as reservoirs for cell media or buffered solution.

The main technical advantage of the organ-on-chip device according to the present invention is that the channels are separated without any membrane between compartments having them one on top of the other.

Furthermore, it can be achieved a semi-circular shape allowing the cell to grow in a 3D environment bearing physiologically relevant features.

This facilitates cell inspection using conventional microscopy or 3D imaging. Also, with this design an organ-on-chip model which is faster, multiplexed, and cheaper than that of the competitors.

### Brief description of the drawings

For better understanding of what has been disclosed, some drawings in which, schematically and only by way of a non-limiting example, a practical case of embodiment is shown.

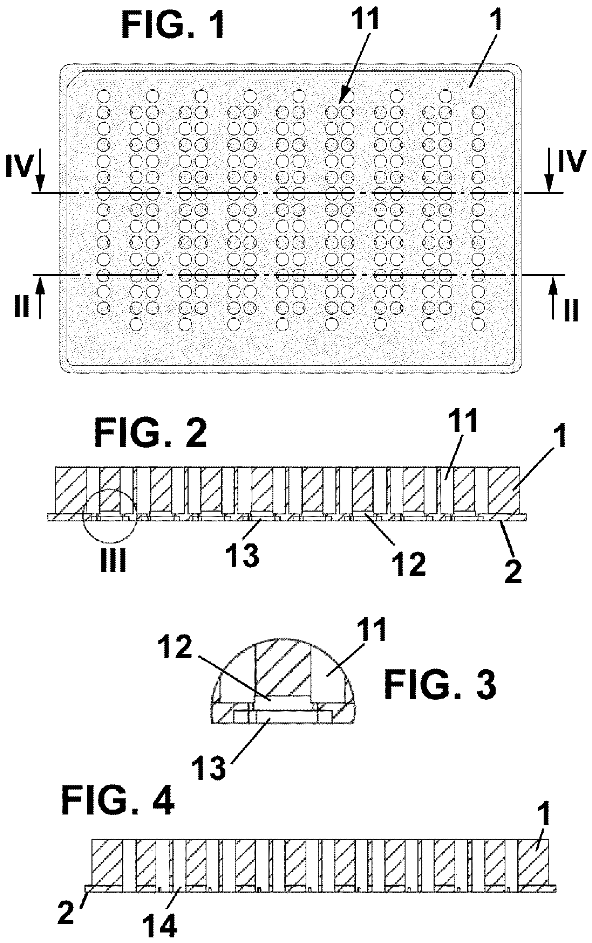

Figure 1 is a top view of the organ-on-chip device according to the present invention;
Figure 2 is a section view along line II-II in Figure 1;
Figure 3 is an enlargement view of the detail III in Figure 2;
Figure 4 is a section view along line IV-IV in Figure 1;

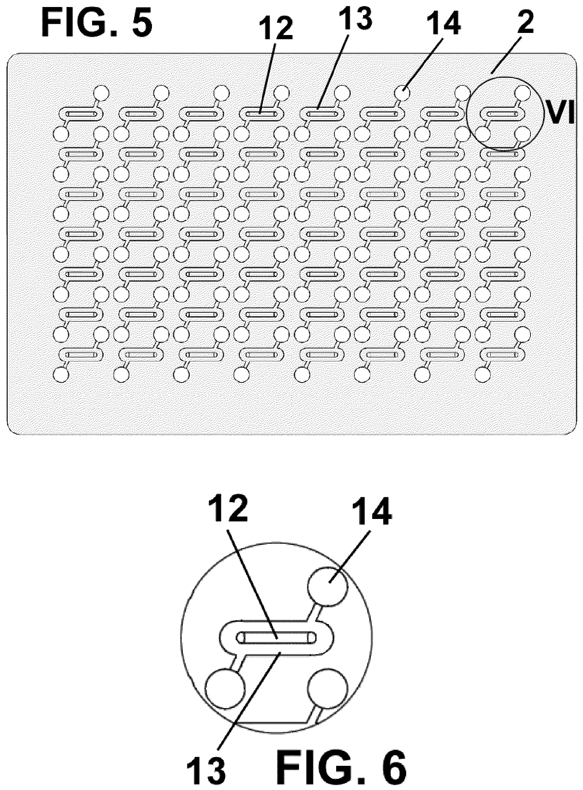

Figure 5 is a bottom view of the organ-on-chip device according to the present invention;
Figure 6 is an enlargement view of the detail VI in Figure 5;
Figure 7 is a perspective view showing the flow of fluid through the channels of the organ-on-chip device according to the present invention; and
Figure 8 is a cross-sectional view of the organ-on-chip device according to the present invention, showing a meniscus that is formed between the channels.

### Description of a preferred embodiment

According to a preferred embodiment, the organ-on-chip device corresponding to the present invention comprises a microfluidic plate (1) comprising a plurality of independent subunits, e.g., 56 subunits, each corresponding to a potential organ-on-a-chip, and a base (2), which preferably includes a flexible polyester film which allows the correct assembly and tightness of the device.

The subunits are preferably disposed according to the ANSI/SLAS Microplate Standards for 384 well microplates. Therefore, the device can be used in standardized equipment such as absorbance, fluorescence and luminescence plate readers, confocal microscopy, multichannel pipettes, etc.

The device is preferably made with transparent materials to allow the optical and continuous monitoring of the organ-on-a-chip subunits.

The microfluidic plate (1) and the base (2) can be assembled using double-sided biocompatible pressure sensitive adhesive, the adhesive being cut following the bottom shape of the microfluidic plate through a cutting machine.

For example, the manufacturing method of the microfluidic plate (1) can be CNC micromachining. This method is ideal for prototypes and high-volume manufacturing of parts in advanced materials such as PMMA (poly dimethyl methacrylate), parts with extremely small tolerances and projects with small or complex designs, e.g., narrow channels or thin walls.

Other manufacturing technologies are also suitable, such as soft-lithography, replica-molding using PDMS (polydimethylsiloxane) or inject molding, that could also be an alternative when the device is wanted to be produced in large volumes.

The organ-on-chip device according to the present invention also comprises channels, each subunit comprising an upper channel (12) and a lower channel (13) are directly overlapped, i.e., there is no physical separation between them, because there is no external membrane. Furthermore, upper channel (12) is connected to two cylindric chambers (11) and lower channel (13) is connected to another two cylindric chambers (14).

Just as an example, each lower channel (13) can have the following dimensions: 10.0 mm length, 1.0 mm height, 3.0 mm width. The volume in each lower channel (13), including adjacent small channels and part of the cylindric chamber (14) can be 65 µL. This volume can be subjected to modifications depending on the fluid used, e.g., in a range of 50-80 µL.

Just as an example, each upper channel (12) can have the following dimensions: 7.0 mm length, 1.5 mm height, 1.0 mm width. The volume in each upper channel (12) can be 10.5 µL.

As stated previously, the ends of the lower (13) channels are independently connected to two cylindric chambers (14), and the ends of upper (12) channels are independently connected to two cylindric chambers (11). Each channel (12, 13) has an inlet chamber and an outlet chamber, and there is a total of four chambers (11, 14) for each organ-on-chip subunit. Besides, these chambers can work as perfusion or as hydrogel hydration.

The ratio of the area between the lower channel (13) and the upper channel (12) at the contact point to each other can also be considered.

This area ratio (α_{A}) can be between 0 to 0.98, regardless of the shape of the lower and upper channels (12, 13).

Just as an example, with the dimensions provided previously, this area ratio (α_{A}) is 0.24 if the area of the lower channel (13) is 7*3+(π *1.5²) = 28.07 mm² and the area of the upper channel (12) is 6*1+(π*0.5²) = 6.79 mm².

Each subunit comprises two overlapping, independent and membrane-free channels (12, 13). The independence of each channel (12, 13) is achieved due to the difference in the contact angle values of the materials used in each part of the device.

For example, if the material of the microfluidic plate (1) is PMMA (poly(methyl methacrylate)), and the water or wettability contact angle is greater than 30°, e.g., 68°. If the material of the microfluidic plate (1) is PDMS (polydimethylsilozane), the water or wettability contact angle is also greater than 30°, e.g., 105°.

For example, if the material of the base (2) is polyester film, the water or wettability contact angle is lower than 90°, e.g., 20°, and if the material of the base (2) is glass, the water or wettability contact angle is also lower than 90°, e.g., 30°. Other materials might be also suitable having into account the condition imposed that this must be more hydrophilic that the microfluidic plate material.

The difference of the water or wettability contact angle between the microfluidic plate (1) and the base (2) allows to introduce a fluid in the lower channel (13) without filling the upper channel (12).

We must indicate that in this description and in the claims, "water contact angle" or "wettability contact angle" is the angle, conventionally measured through the liquid, where a liquid-vapor interface meets a solid surface. It quantifies the wettability of a solid surface by a liquid. A given system of solid, liquid, and vapor at a given temperature and pressure has a unique equilibrium contact angle. The contact angle depends upon the medium above the free surface of the liquid, and the nature of the liquid and solid in contact, and it is independent of the inclination of solid to the liquid surface.

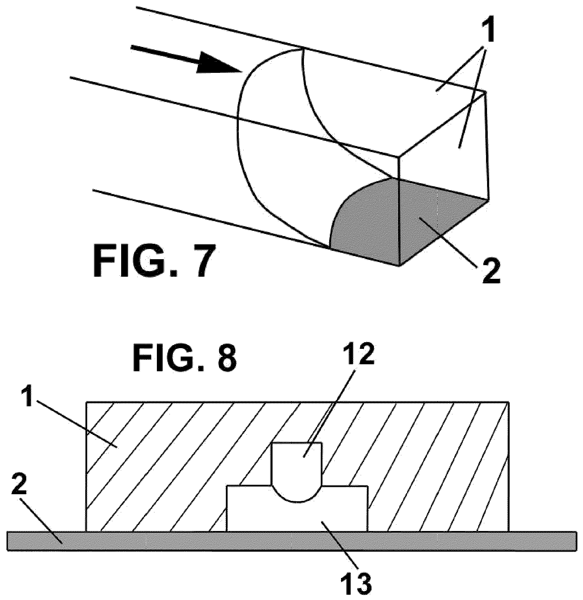

The upper channel (12) tends to repel the fluid coming from the lower channel (13) providing an air gap. Fig. 7 shows the irregular flow in a rectangular microchannel with heterogeneous contact angles for the base (2) and the microfluidic plate (1).

Fig. 8 shows that when fluid from the lower channel (13) is filled, a meniscus can be appreciated. This meniscus separates the lower (13) and the upper (12) channels.

Just as an example, collagen (unpolymerized) can be used as fluid for the lower channel (13). Once collagen is put in the incubator, e.g., at 37°C, this polymerizes in a form of hydrogel. Once the fluid in the lower channel (13) is polymerized, the upper channel (12) can be filled with a second fluid, e.g., cell medium. This creates two overlapping channels (12, 13) without the need of an external membrane.

One example of the function of the organ-on-chip device according to the present invention is described hereinafter.

As stated previously, the lower channel (13) can be used with a collagen gel, as it allows a fast polymerization at 37 °C. This gel represents the extracellular matrix (ECM) of the cells. ECM is a three-dimensional network that encompasses all the tissues and cells of the body. It constitutes a structural support that allows cells to differentiate into specific cell lines giving a specific morphology and function. In addition, it also allows the transmission of mechanical forces towards the basement membrane, and acts as a biophysical filter for protection, nutrition, and cell innervation.

The ECM is made up of proteins, glycosaminoglycans, proteoglycans and glycoproteins, collagen being one of the most important proteins in its correct function and structure. The reservoir of the ECM channel can be filled with media to keep the hydrogel hydrated.

The upper channel (12) is normally filled with culture media to provide nutrients, and for generating perfusing. There is no need to use any pumps or tubes, as the microfluidic flow of fresh media is done by gravitational forces.

Calculating the flow and the stress caused by it due to the shear stress, an inclination is provided, e.g., of 5° for 8 min, per side. With a rocker programmed at these specifications, a continuous and bidirectional flow is achieved. This allows gravitational perfusion of the culture medium without the need to use connectors or tubes.

For a more complete disclosure of the organ-on-chip device of the present invention, some examples of use are described hereinafter.

The organ-on-chip device according to the present invention can be used as Gut-on-a-chip, Cartilage-on-chip, Tendon-on-chip, Tumor-on-chip models in order to study drug screening, toxicity studies, material screening, chemicals screening, etc.

For Cartilage-on-a-chip and Tendon-on-a-chip, the cells are embedded in an extracellular matrix (ECM) of collagen I, and they are subsequently introduced in the lower channel prior to polymerize. Once the collagen polymerizes, the culture medium is introduced in the upper channel to feed the cells. Collagen I matrix polymerizes at 37°C after 20-30 minutes.

For Gut-on-a-chip and Tumor-on-a-chip, the extracellular matrix (ECM) of collagen I is introduced in the lower channel, and subsequently polymerized at 37 °C for 20-30 minutes. Once the gel is polymerized, epithelial cells or organoids are seeded in the upper channel in order to grow on the gel surface.

Although reference has been made to specific embodiments of the invention, it is apparent to a person skilled in the art that the described organ-on-chip device is susceptible of numerous variations and modifications, and that all the details mentioned can be replaced by other technically equivalents, without departing from the scope of protection defined by the appended claims.

## Claims

1. Organ-on-chip device, comprising a microfluidic plate (1), a base (2), and a plurality of independent subunits, each subunit comprising an upper channel (12) and a lower channel (13), said channels (12, 13) being defined between the microfluidic plate (1) and the base (2), **characterized in that** the microfluidic plate (1) has a wettability contact angle greater than 30°, and the base (2) has a wettability contact angle lower than 90°.

2. Organ-on-chip device according to claim 1, wherein the microfluidic plate (1) is made of poly(methyl methacrylate) and the wettability contact angle between 60° and 75°.

3. Organ-on-chip device according to claim 1, wherein the microfluidic plate (1) is made of polydimethylsilozane and the wettability contact angle is between 100° and 110°.

4. Organ-on-chip device according to any one of the previous claims, wherein the base (2) is made of polyester film and the wettability contact angle is between 15° and 25°.

5. Organ-on-chip device according to any one of claims 1-3, wherein the base (2) is made of glass and the wettability contact angle is between 25° and 35°.

6. Organ-on-chip device according to any one of the previous claims, wherein the material base (2) is more hydrophilic than the material of the microfluidic plate (1).

7. Organ-on-chip device according to any one of the previous claims, wherein the upper and lower channels (12, 13) of each subunit are overlapped to each other.

8. Organ-on-chip device according to claim 7, wherein the upper and lower channels (12, 13) of each subunit are directly overlapped to each other.

9. Organ-on-chip device according to any one of the previous claims, wherein each of the upper and lower channels (12, 13) of each subunit define an area at the contact point to each other, the ratio of the area between the lower channel (13) and the upper channel (12) being between 0 and 0.98.

10. Organ-on-chip device according to claim 9, wherein the ratio of the area between the lower channel (13) and the upper channel (12) is between 0.15 and 0.30.

11. Organ-on-chip device according to any one of the previous claims, wherein each channel (12, 13) is connected to two cylindric chambers (11, 14), respectively.
